(19) Europäisches Patentamt
European Patent Office
Office européen des brevets

(11) Publication number: **0 111 990 B1**

(12) **EUROPEAN PATENT SPECIFICATION**

(45) Date of publication of patent specification: **07.10.87**

(21) Application number: **83304341.7**

(22) Date of filing: **27.07.83**

(51) Int. Cl.⁴: **C 07 D 471/04,** A 61 K 31/47 // (C07D471/04, 231:00, 221:00)

(54) **Hypotensive agents.**

(30) Priority: **03.11.82 US 438833**
**03.11.82 US 439238**

(43) Date of publication of application:
**27.06.84 Bulletin 84/26**

(45) Publication of the grant of the patent:
**07.10.87 Bulletin 87/41**

(84) Designated Contracting States:
**AT BE CH DE FR GB IT LI NL SE**

(58) References cited:
**EP-A-0 013 787**

(73) Proprietor: **ELI LILLY AND COMPANY**
**307, East McCarty Street**
**Indianapolis Indiana 46285 (US)**

(72) Inventor: **Hahn, Richard Allen**
**7819 Teel Way**
**Indianapolis Indiana 46256 (US)**
Inventor: **Titus, Robert Daniel**
**3818 Spann Avenue**
**Indianapolis Indiana 46203 (US)**
Inventor: **Kornfeld, Edmund Carl**
**5159 East 76th Court**
**Indianapolis Indiana 46250 (US)**

(74) Representative: **Crowther, Terence Roger et al**
**Erl Wood Manor**
**Windlesham Surrey GU20 6PH (GB)**

**EP 0 111 990 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel hypotensive agents and is also concerned with novel methods of preparing those compounds.

There are presently a number of commercial products available which are to a greater or lesser extent effective in reducing elevated blood pressure in humans, see, for instance, U.S. Patent No. 2,484,029 which describes the commercial product "Hydralazine", 1(2H)-phthalazinone hydrazone. However, these products all have various undesirable side-effects, for instance, postural hypotensive effects and tachycardia. Accordingly, there continues to be a need for new hypotensive agents possessing fewer undesirable side-effects.

Surprisingly, in accordance with the present invention, it has been discovered that a stereoisomer of the racemic form of the compound described in EP—A—0 013 787 and U.S. Patent No. 4,198,415, see Example 2, possesses extremely interesting hypotensive properties. It is to be noted that this U.S. Patent teaches that such compounds are dopamine agonists useful as prolactin inhibitors and in the treatment of Parkinson's disease, a utility far removed from that now found for the novel isomer provided herein.

Thus, in accordance with the invention the stereoisomer illustrated below in the form of its tautomers:

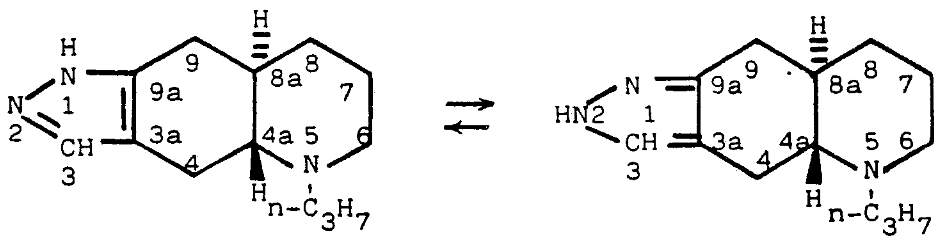

Ia Ib

is an effective hypotensive agent.

The above stereoisomer can be alternatively designated as: 5 - n - propyl - 4,4aβ,5,6,7,8,8aα,9 - octahydro - 1H(and 2H)pyrazolo[3,4 - g]quinoline; as trans - (−) - 5 - n - propyl - 4,4a,5,6,7,8,8a,9 - octahydro - 1H - (and 2H)pyrazolo[3,4 - g]quinoline as 4aR,8aR - 5 - n - propyl - 4,4a,5,6,7,8,8a,9 - octahydro - 1H(and 2H)pyrazolo[3,4 - g]quinaoline; or as trans - 4aR - 5 - n - propyl - 4,4a,5,6,7,8,8a,9 - octahydro - 1H(and 2H)pyrazolo[3,4 - g]quinoline.

This invention also provides a novel method of blocking norepinephrine release from peripheral sympathetic nerve terminals that innervate arterial muscle fibres in mammals which comprises administering to a mammal having an elevated blood pressure an effective dose of a tautomeric mixture of trans - 4aR - 5 - n - propyl - 4,4a,5,6,7,8,8a,9 - octahydro - 1H(and 2H) - pyrazolo[3,4 - g]quinoline, represented by formula Ia or Ib above, or a pharmaceutically-acceptable salt thereof, thereby lowering the blood presure of said mammal.

The above tautomeric pair (Ia and Ib) can be prepared in optically pure form by:

(a) reacting trans - dl - 5 - n - propyl - 4,4a,5,6,7,8,8a,9 - octahydro - 1H(and 2H)pyrazolo[3,4 - g]-quinoline, with an excess of an optically active tartaric acid of the formula

$$HOOC—(CHOR^2)_2—COOH \qquad II$$

wherein $R^2$ is hydrogen or

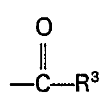

where $R^3$ is $C_1$—$C_3$ alkyl, phenyl, or substituted phenyl;

(b) separating the tartrate salt of the trans-stereoisomer, thus formed;

(c) fractionally recrystallizing the tartrate salt mixture to provide the desired trans-4aR tartrate salt;

(d) reacting an aqueous solution of the tartrate salt with excess base; and then

(e) isolating the trans-4aR-5-n-propyl-4,4a,5,6,7,8,8a,9-octahydro-1H(and 2H)pyrazolo[3,4-g]quinoline free base thus formed; and

(f) optionally salifying the free base so obtained so as to obtain a pharmaceutically-acceptable salt thereof.

An optically active tartaric acid of the formula

$$HOOC—(CHOR^2)_2—COOH \qquad II$$

wherein $R^2$ is hydrogen or

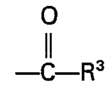

where $R^3$ is $C_1$—$C_3$ alkyl, or phenyl optionally substituted by groups such as methyl, chloro or methoxy, is used in the above process. The acid will of course be in the (D)-(−) form. A preferred class of reagents of formula II are those where $R^2$ is hydrogen, $R^3$ is methyl, phenyl or *p*-tolyl. An especially preferred reagent of formula II is D-(−)-S-tartaric acid. The reaction can be effected using a solvent, such as a $C_1$—$C_4$ alkanol, preferably methanol, and at a temperature from about 50°C to reflux.

Conversion of the salt, e.g. pyrazolo[3,4-g]quinoline D-(−)-S-tartrate, to the free base is readily effected by dissolving the salt in water and then adding an excess of a base (NaOH, $Na_2CO_3$, $NH_4OH$). The pyrazolo-[3,4-g]quinoline, being insoluble in the basic solution, separates and is extracted with a water-immiscible organic solvent. The organic layer is separated and dried. If it is desired to prepare a different salt, a solution containing one equivalent of a second non-toxic acid can then be added, and the salt isolated by filtration or evaporation. Alternatively, the solvent can be removed form the dried organic extract and the free base obtained as a residue. The free base can then be dissolved in a suitable solvent and the different non-toxic acid added as a solution.

Compounds according to the above structure contain two basic groups, the alkylated quinoline ring nitrogen and the pyrazole nitrogen not carrying the hydrogen (N—1 or N—2). The quinoline ring nitrogen is the more basic of the two and forms acid addition salts readily. Strong inorganic acids such as the mineral acids or strong organic acids such as *p*-toluenesulfonic acid, can form di-salts when employed in excess.

Pharmaceutically-acceptable salts of the compounds of formula Ia or Ib thus include mono- or di-salts derived from inorganic acids such as: hydrochloric acid, nitric acid, phosphoric acid, sulfuric acid, hydrobromic acid, hydriodic acid, phosphorous acid and the like, as well as salts derived from organic acids such as aliphatic mono- and dicarboxylic acids, phenyl-substituted alkanoic acids, hydroxy alkanoic and alkandioic acids, aromatic acids, aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate, naphthalene-2-sulfonate and the like salts.

The preferred salt of this invention is the mono HCl salt and can be prepared for example by adding an equivalent of hydrogen chloride to an ethanol solution of the free base, followed by evaporation of the ethanol and recrystallization of the salt. If it is desired to make a disalt such as a dihydrochloride salt, HCl gas can be passed into a solution of the free base to the point of saturation and the salt isolated by filtration.

The following examples further illustrate the preparation of the compounds of the invention.

### Example 1

One equivalent of trans-dl-5-n-propyl-4,4a,5,6,7,8,8a,9-octahydro-1H(and 2H)pyrazolo[3,4-g]quinoline was dissolved in methanol. One and one-tenths equivalents of D-(−)-S-tartaric acid were added thereto and the solution heated to boiling for about 10 minutes. Upon cooling to room temperature, a crystalline precipitate formed which was separated by filtration. Five-fold recrystallization of that precipitate from methanol (0.1 g of tartrate salt per 1 ml of methanol) yielded crystalline trans-4aR-5-n-propyl-4,4a,5,6,7,8,8a,9-octahydro-1H(and 2H) pyrazolo[3,4-g]quinoline, D-(−)-S-tartrate melting at 201—202°C $[\alpha]_D^{25}$ ($H_2O$) = −95.5°. The salt was of satisfactory optical purity since the last recrystallization did not substantially change the rotation of plane polarized light.

The above salt was dissolved in water and the aqueous solution made strongly basic with 14N aqueous ammonium hydroxide. The alkaline aqueous layer was extracted three times with an equal volume of a 3:1 chloroform:isopropanol solvent mixture. The organic extracts were combined, the combined extracts washed with saturated aqueous sodium chloride and then dried. Evaporation of the solvent yielded a colorless solid comprising trans-4aR-5-n-propyl-4,4a,5,6,7,8,8a,9-octahydro-1H(and 2H) pyrazolo[3,4-g]-quinoline. This solid was dissolved in methanol and precisely one equivalent of 0.2N aquoeus hydrochloric acid added. The volatile constituents were removed by evaporation *in vacuo* and the resulting hydrochloride salt crystallized from a methanol/ether solvent mixture. Trans-4aR-5-n-propyl-4,4a,5,6,7,8,8a,9-octahydro-1H(and 2H) pyrazolo[3,4-g]quinoline, hydrochloride thus prepared melted at about 280°C with decomposition; $[\alpha]_D^{25}$ ($H_2O$) = −120.6°.

| | | | | |
|---|---|---|---|---|
| Analysis Calculated: | C, 61.04; | H, 5.67; | Cl, 13.86; | N, 16.43, |
| Found: | C, 61.30; | H, 5.51; | Cl, 14.14; | N, 16.62. |

The inactive trans-(+)- or trans-4aS-isomer is prepared by contacting the trans-dl-racemate with a slight excess of L-(+)-R-tartaric acid.

The absolute configuration of the inactive trans-(+) stereoisomer was shown by x-ray analysis of its D-(−)-R-*t*-butyloxycarbonylphenylglycine salt to be 4aS-configuration. Thus, the active isomer is, by process of elimination, the trans 4aR, or 4aβ,8aα, or 4aR,8aR or trans-(−)-isomer.

This invention also concerns a pharmaceutical composition comprising as active ingredient a tautomer of formula Ia or Ib as defined above, or a pharmaceutically-acceptable salt thereof, associated with a pharmaceutically-acceptable carrier therefor.

In using the therapeutic process, a pharmaceutically-acceptable salt of a drug according to formula Ia or Ib above formed with a non-toxic acid is administered orally or parenterally to a mammal with an elevated blood pressure in which it is desired to lower blood pressure, by blocking norepinephrine release from peripheral sympathetic nerve terminals that innervate arterial muscle fibers. For parenteral administration, a water soluble salt of the drug, trans-4aR-5-n-propyl-4,4a,5,6,7,8,8a,9-octahydro-2H(and 1H) pyrazolo[3,4-g]isoquinoline, is dissolved in an isotonic salt solution and administered by the I.V. route. Dose levels of from 0.5—500 mcg./kg. of mammalian weight are found to be effective to block norepinephrine release and thereby reduce elevated blood pressure in spontaneously hypertensive rats (SHR). For oral administration, a pharmaceutically-acceptable salt of the drug is mixed with standard pharmaceutical excipients such as starch and loaded into capsules, each containing 0.1—15 mg. of active drug. Dosage levels of from 0.1—3 mg./kg. have been found to be effective in blocking norepinephrine release from sympathetic nerve terminals, thereby lowering blood pressure in SHR, for periods ranging up to six hours. Thus, the oral dosage would be administered 3—4 times per day, giving a daily dosage range of about 0.1 to about 15 mg./kg. per day.

Other oral dosage forms, suspension, elixers and tablets, can also be utilized and are prepared by standard procedures.

The effect of the method of this invention in blocking norepinephrine release from peripheral sympathetic nerve terminals that innervate arterial muscle fibers, thereby reducing the blood pressure in spontaneously hypertensive rats, is shown by the following experiment:

Adult male spontaneously hypertensive rats (SHR) (Taconic Farms, Germantown, New York), weighing approximately 300 g. were anesthetized with pentobarbital sodium (60 mg./kg., i.p.). The trachea was cannulated and SHR respired room air. Pulsatile arterial blood pressure was measured from a cannulated carotid artery using a Statham transducer (P23 ID). Mean arterial blood pressure was calculated as diastolic blood pressure plus 1/3 pulse pressure. Cardiac rate was monitored by a cardiotachometer which was triggered by the systolic pressure pulse. Drug solutions were administered I.V. through a catheter placed in a femoral vein. ARterial blood pressure and cardiac rate were recorded on a multichannel oscillograph (Beckman, Model R511A). Fifteen minutes were allowed to elapse following surgery for equilibration of the preparation.

Table 1 which follows gives the results of this experiment using trans-4aR-5-n-propyl-4,4a,5,6,7,8,8a,9-octahydro-2H(and 1H) pyrazolo[3,4-g]quinoline, D-(−)-S-tartrate, plus the corresponding trans-4aS-enantiomer and the parent trans-dl-racemate as the hydrochloride salt for comparison purposes. Each drug was administered I.V. to groups of four SHR at a series of dose levels.

TABLE 1

Relative potencies of trans-5-n-propyl-4,4a,5,6,7,8,8a,9-octahydro-2H(and 1H)pyrazolo[3,4-g]-quinolines isomers and racemate.

| Compound | Dose mcg./kg. | Mean Arterial Blood Pressure % Change (±) |
|---|---|---|
| Trans-4aR | 0.1 | − 7.7 ± 1.6 |
| | 1 | +14.3 ± 1.6 |
| | 10 | −25.2 ± 2.1 |
| | 100 | −38.6 ± 4.3 |
| Trans-4aS | 0.1 | − 8.5 ± 0.8 |
| | 1 | − 5.1 ± 0.8 |
| | 10 | − 5.0 ± 0.3 |
| | 100 | − 4.6 ± 0.3 |
| | 1000 | − 6.4 ± 1.1 |
| Trans-dl | 1 | −12.7 ± 2.2 |
| | 10 | −22.4 ± 0.7 |
| | 100 | −32.0 ± 2.1 |
| | 1000 | −52.2 ± 6.9 |

It is apparent that all the hypotensive action of trans-dl-5-n-propyl-4,4a,5,6,7,8,8a,9-octahydro-1H(and 2H)-pyrazolo[3,4-g]quinoline resides in the levo or trans-4aR stereoisomer.

More direct evidence than trans-dl-5-n-propyl-4,4a,5,6,7,8,8a,9-octahydro-1H(and 2H)-pyrazolo[3,4-g]-quinoline blocks norepinephrine release from peripheral sympathetic nerve terminals is illustrated by the following experiment. In this experiment, SHR were pithed by passing a steel rod through the right orbit and down the entire length of the spinal column. The rod was left in position for the duration of the experiment. Immediately after pithing, SHR were ventilated with room air delivered from a rodent respirator (Harvard, Model 680; tidal volume of 1 ml./100 g. body weight, 60 cycles/min). The pithing rod was used to stimulate the entire sympathetic outflow from the spinal cord. Portions of the rod which lay in the cervical and sacral cord were insulated. The sympathetic outflow was stimulated by square waved pulses (50 volts, 1 msec duration for 30 sec) of frequencies of 0.25, 1, 4 and 8 Hz delivered from a stimulator (Grass, Model S44). The pithing rod served as the stimulating electrode while a needle inserted into the right hind-limb musculature was the indifferent electrode. Skeletal muscle twitches were prevented by administration of d-tubocurarine (1 mg./kg., I.V.). Increments in diastolic blood pressure produced by electrical stimulation of the sympathetic outflow were monitored from a cannulated carotid artery. The test drug was administered I.V. at two dose levels to groups of four SHR.

Table 2 which follows indicates that electrical stimulation of the sympathetic outflow or I.V. administration of exogenous norepinephrine each produced the expected vasconstrictor responses in control pithed SHR, as indicated by the increments in diastolic blood pressure. Pretreatment of other SHR with the trans-dl-racemate attenuated neurogenic vasoconstrictor responses in a dose-related manner. This attenuation was selective in that the test drug produced no comcomitant antagonism of comparable vasoconstrictor responses resulting from administration of exogenous norepinephrine. Thus, the composite data of Tables 1—2 indicate that doses of the trans-dl-racemate (and by implication, of the trans-4aR-isomer, the active component of the racemate) which are hypotensive in intact SHR, result in selective inhibition of norepinephrine release from peripheral sympathetic nerve terminals.

TABLE 2

Selective antagonism of neurogenic vasconstrictor responses produced by trans-dl-5-n-propyl-4,4a,5,6,7,8,8a,9-octahydro-1H(and 2H)-pyrazolo[3,4-g]quinoline.

| Trans-dl-racemate (mcg./kg., I.V.) | Increase in Diastolic Blood Pressure (mm. Hg.) Produced by Electrical Stimulation of the Sympathetic Outflow | | | |
|---|---|---|---|---|
| | 0.25 Hz | 1 Hz | 4 Hz | 8 Hz |
| none | 34 ± 3 | 72 ± 3 | 118 ± 8 | 129 ± 7 |
| 100 | 14 ± 2 | 47 ± 2 | 105 ± 8 | 115 ± 9 |
| 1000 | 10 ± 1 | 26 ± 4 | 65 ± 8 | 89 ± 12 |

| Trans-dl-racemate (mcg./kg., I.V.) | Increase in Diastolic Blood Pressure (mm. Hg.) Produced by Exogenous Norepinephrine | | | |
|---|---|---|---|---|
| | 0.01 mcg./kg. | 0.1 mcg./kg. | 1 mcg./kg. | 10 mcg./kg. |
| none | 8 ± 1 | 27 ± 1 | 78 ± 3 | 140 ± 5 |
| 100 | 7 ± 1 | 29 ± 2 | 74 ± 1 | 128 ± 5 |
| 1000 | 10 ± 1 | 30 ± 2 | 80 ± 1 | 140 ± 4 |

The lack of alpha adrenergic receptor activity for trans-4aR-5-n-propyl-4,4a,5,6,7,8,8a,9-octahydro-1H(and 2H)-pyrazolo[3,4-g]quinoline is illustrated by the following experiment in which vasoconstrictor activity (or lack thereof) can be demonstrated in pithed SHR as a rise in baseline blood pressure, vasoconstrictor activity resulting in a blood pressure rise.

The effects on blood pressure as a measure of the vasoconstrictor effects of trans-dl-5-n-propyl-4,4a,5,6,7,8,8a,9-octahydro-1H(and 2H)-pyrazolo[3,4-g]quinoline and the trans-4aR stereoisomer were determined using norepinephrine as a control substance. Two other dopamine agonists, pergolide and lergotrile, were also included plus the 7-methylmercaptomethyl derivative of trans-dl-5-n-propyl-4,4a,5,6,7,8,8a,9-octahydro-1H(and 2H)-pyrazolo[3,4-g]quinoline. Table 3 which follows gives the results of this comparison. In the table, column 1, gives the name of the test compound, column 2, the dose and column 3, the change in diastolic blood pressure. Four pithed SHR rats were used at each dose level for each drug.

TABLE 3

Relative blood pressure effects of trans-dl-5-n-propyl-4,4a,5,6,7,8,8a,9-octahydro-1H(and 2H)-pyrazolo-[3,4-g]quinoline, the trans-4aR stereoisomer and related compounds.

| Compound | IV Dose in mcg./kg. | Change in Distolic Blood Pressure (mm. Hg.) |
|---|---|---|
| Trans-dl | 1 | + 5 ± 1 |
| | 10 | + 2 ± 3 |
| | 100 | + 2 ± 4 |
| | 1000 | + 7 ± 1 |
| Trans-4aR | 1 | + 4 ± 1 |
| | 10 | −5 ± 1 |
| | 100 | −4 ± 1 |
| | 1000 | − 3 ± 1 |
| Norepinephrine | .01 | + 8 ± 1 |
| | .1 | +27 ± 1 |
| | 1 | +78 ± 3 |
| | 10 | +140 ± 5 |
| Pergolide | 1 | + 4 |
| | 10 | +19 |
| | 100 | +62 |
| Lergotrile | 10 | + 6 |
| | 100 | + 6 |
| | 1000 | +18 |
| Trans-dl-5-n-propyl-7-methylmercaptomethyl-4,4a,5,6,7-8,8a,9-octahydro-1H-(and 2H)-pyrazolo(3,4-g]quinoline | 1 | + 2 ± 2 |
| | 10 | + 5 ± 1 |
| | 100 | +17 ± 1 |
| | 1000 | +49 ± 1 |

From the above data, it would also be expected that the trans-4aR steroisomer would not increase cardiac rate as does norepinephrine and this hypothesis has been affirmed by experiment.

It is apparent from the data presented in Tables 2—3 that trans-4aR-5-n-propyl-4,4a,5,6,7,8,8a,9-octahydro-1H(and 2H)-pyrazolo[3,4-g]quinoline and the trans-dl racemate are neither an α-receptor agonists nor antagonists, that their hypotensive action is not caused by any α-receptor effect, but is probably attributable solely to their ability to block norepinephrine release from peripheral sympathetic nerve terminals that innervate arterial muscle fiber.

Trans-4aR-5-n-propyl-4,4a,5,6,7,8,8a,9-octahydro-1H-(and 2H)quinoline can be considered as related to the ergoline part structure (IV) except that the pyrrole function is replaced by a pyrazole function (Va and Vb).

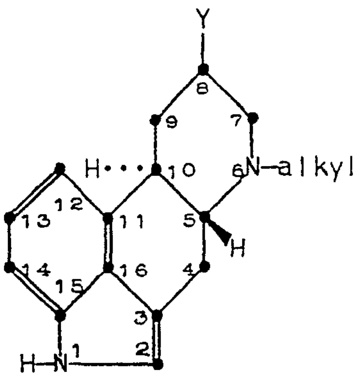

ergoline

III

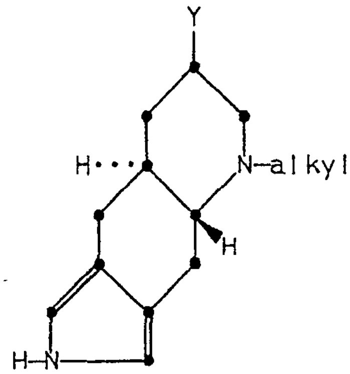

IV

where y is H and alkyl is $CH_3$

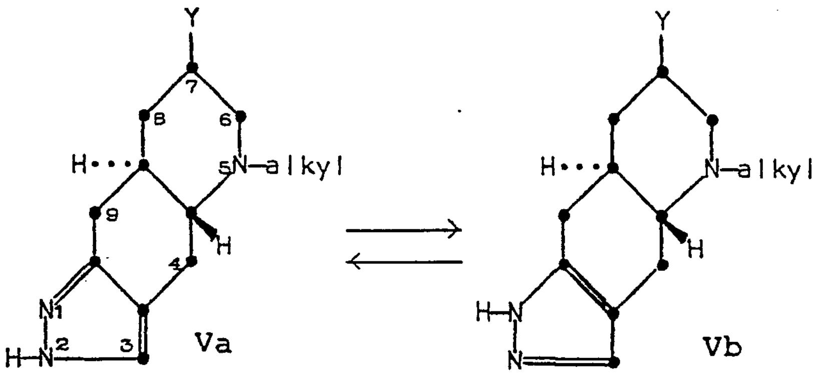

Va

Vb

where Y is H and alkyl is n-propyl.

Pergolide (see Table 2) is an ergoline where alkyl is n-propyl and Y is methylmercaptomethyl. Lergotrile (from Table 2) is an ergoline where alkyl is methyl, Y is $CH_2CN$ and there is a chlorine at 2. The related compound trans-dl-5-n-propyl-7-methylmercaptomethyl-4,4a,5,6,7,8,8a,9-octahydro-1H(and 2H)-pyrazolo[3,4-g]quinoline (Va and Vb where alkyl is n-propyl and Y is methylmercaptomethyl), is disclosed in U.S. Patent 4,198,415, and is a substituted derivative of the compound used in the process of this invention, the trans-4aR derivative, Va and Vb where alkyl is also n-propyl but Y is H. From the data in Table 2, it can be seen that pergolide and trans-dl-5-n-propyl-7-methylmercaptomethyl-4,4a,5,6,7,8,8a,9-octahydro-1H(and 2H)-pyrazolo[3,4-g]quinoline behave like norepinephrine in the pithed SHR. Thus, while these later compounds and dopamine agonists, they also act on α-receptors. Lergotrile, while it resembles the trans-4aR isomer useful in the process of this invention in that it does not act as a vascoconstrictor in pithed SHR, has undesirable central effects such as a potential for producing hallucinations in humans and is thus not a pure presynaptic dopamine agonist as is the trans-4aR isomer. The trans-4aR isomer is apparently unique as a presynaptic dopamine agonist. As aconsequence, it is unique in its ability to lower an elevated blood pressure in mammals by blocking norepinephrine release from peripheral sympathetic nerve terminals that innervate arterial muscle fibers without other major pharmaceutical activity.

**Claims for the Contracting States: BE CH DE FR GB IT LI LU NL SE**

1. Trans-4aR-5-n-propyl-4,4a,5,6,7,8,8a,9-octahydro-1H(and 2H)-pyrazolo[3,4-g]quinoline of formula Ia or Ib:

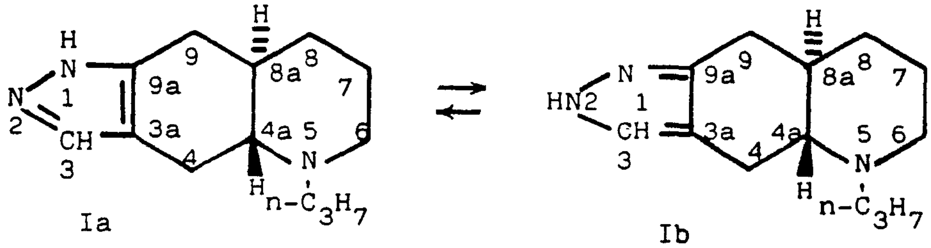

Ia

Ib

or a pharmaceutically-acceptable salt thereof.

2. Trans-4aR-5-n-propyl-4,4a,5,6,7,8,8a,9-octahydro-1H(and 2H)-pyrazolo[3,4-g]quinoline.

3. Trans-4aR-5-n-propyl-4,4a,5,6,7,8,8a,9-octahydro-1H(and 2H)-pyrazolo[3,4-g]quinoline, hydrochloride.

4. A pharmaceutical composition comprising as active ingredient a tautomer of formula Ia or Ib as defined in claim 1, or a pharmaceutically-acceptable salt thereof, associated with a pharmaceutically-acceptable carrier therefor.

5. A tautomer of formula Ia or Ib, or a pharmaceutically-acceptable salt thereof, as claimed in any one of claims 1 to 3, for use as a hypotensive agent.

6. A process for preparing a tautomer of formula Ia or Ib, or a pharmaceutically-acceptable salt thereof, as claimed in any one of claims 1 to 3, which comprises:

(a) reacting trans - dl - 5 - n - propyl - 4,4a,5,6,7,8,8a,9 - octahydro - 1H(and 2H)pyrazolo[3,4 - g]-quinoline, with an excess of an optically active tartaric acid of the formula

$$HOOC—(CHOR^2)_2—COOH \qquad II$$

wherein $R^2$ is hydrogen or

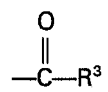

where $R^3$ is $C_1$—$C_3$ alkyl, phenyl, or substituted phenyl;

(b) separating the tartrate salt of the trans-stereoisomer, thus formed;

(c) fractionally recrystallizing the tartrate salt mixture to provide the desired trans-4aR tartrate salt;

(d) reacting an aqueous solution of the tartrate salt with excess base; and then

(e) isolating the trans-4aR-5-n-propyl-4,4a,5,6,7,8,8a,9-octahydro-1H(and 2H)pyrazolo[3,4-g]quinoline free base thus formed; and

(f) optionally salifying the free base so obtained so as to obtain a pharmaceutically-acceptable salt thereof.

**Claims for the Contracting State: AT**

1. A process for preparing a tautomer of formula Ia or Ib:

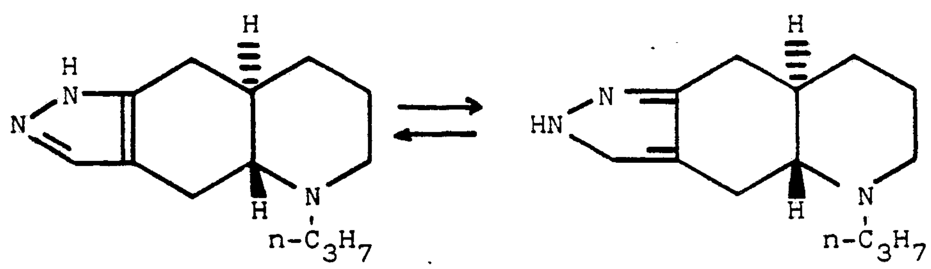

Ia Ib

or a pharmaceutically-acceptable salt thereof, which comprises:

(a) reacting trans - dl - 5 - n - propyl - 4,4a,5,6,7,8,8a,9 - octahydro - 1H(and 2H)pyrazolo[3,4 - g]-quinoline, with an excess of an optically active tartaric acid of the formula

$$HOOC—(CHOR^2)_2—COOH \qquad II$$

wherein $R^2$ is hydrogen or

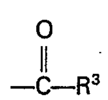

where $R^3$ is $C_1$—$C_3$ alkyl, phenyl, or substituted phenyl;

(b) separating the tartrate salt of the trans-stereoisomer, thus formed;

(c) fractionally recrystallizing the tartrate salt mixture to provide the desired trans-4aR tartrate salt;

(d) reacting an aqueous solution of the tartrate salt with excess base; and then

(e) isolating the trans-4aR-5-n-propyl-4,4a,5,6,7,8,8a,9-octahydro-1H(and 2H)pyrazolo[3,4-g]quinoline free base thus formed; and

(f) optionally salifying the free base so obtained so as to obtain a pharmaceutically-acceptable salt thereof.

2. The process of claim 1 in which the hydrochloride salt is formed.

3. The process of claim 1 or 2 wherein the optically active tartaric acid of formula II is one where $R^2$ is hydrogen, or $R^3$ is methyl, phenyl or *p*-tolyl.

4. The process of claim 3 wherein the optically active tartaric acid of formula II is D-(−)-S-tartaric acid.

5. A process of claim 1 wherein a $C_1$—$C_4$ alkanol solvent is present.

6. A tautomer of formula Ia or Ib whenever prepared by a process according to any one of claims 1 to 5.

**Patentansprüche für die Vertragsstaaten: BE CH DE FR GB IT LI NL SE**

1. Trans-4aR-5-n-propyl-4,4a,5,6,7,8,8a,9-octahydro-1H(und 2H)pyrazol[3,4-g]chinolin der Formeln Ia oder Ib

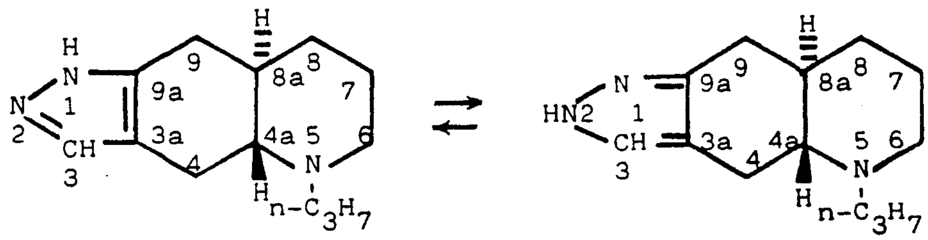

Ia Ib

oder ein pharmazeutisch annehmbares Salz hiervon.

2. Trans-4aR-5-n-propyl-4,4a,5,6,7,8,8a,9-octahydro-1H(und 2H)pyrazol[3,4-g]chinolin.

3. Trans-4aR-5-n-propyl-4,4a,5,6,7,8,8a,9-octahydro-1H(und 2H)pyrazol[3,4-g]chinolinhydrochlorid.

4. Pharmazeutische Zusammensetsung, dadurch gekennzeichnet, daß sie Tautomeres der Formeln Ia oder Ib gemäß Anspruch 1 oder ein pharmazeutisch annehmbares Salz hiervon als Wirkstoff in Verbindung mit einem pharmazeutisch annehmbaren Träger hierfür enthält.

5. Verwendung eines Tautomeren der Formeln Ia oder Ib oder eines pharmazeutisch annehmbaren Salzes hiervon gemäß irgendeinem der Ansprüche 1 bis 3 als hypotensives Mittel.

6. Verfahren zur Herstellung eines Tautomeren der Formeln Ia oder Ib oder eines pharmazeutisch annehmbaren Salzes hiervon gemäß irgendeinem der Ansprüche 1 bis 3, dadurch gekennzeichnet, daß man

(a) trans-dl-5-n-propyl-4,4a,5,6,7,8,8a,9-octahydro-1H(und 2H)pyrazol[3,4-g]chinolin mit einem Überschuß einer optisch aktiven Weinsäure der Formel

$$HOOC—(CHOR^2)_2—COOH \quad \text{(II)}$$

worin $R^2$ Wasserstoff oder

$$-\overset{\overset{\displaystyle O}{\|}}{C}-R^3 \text{ ist,}$$

wobei $R^3$ für $C_1$—$C_3$-Alkyl, Phenyl oder substituiertes Phenyl steht, umsetzt,

(b) das Tartratsalz des so gebildeten trans-Stereoisomeren abtrennt,

(c) das Tartratsalzgemisch durch fraktionierte Umkristallisation in das gewünschte trans-4aR-Tartratsalz überführt,

(d) eine wäßrige Lösug des Tartratsalzes mit einem Überschuß an Base umsetzt, und

(e) die so gebildete freie Base von trans-4aR-5-n-propyl-4,4a,5,6,7,8,8a,9-octahydro-1H(und 2H)pyrazol-[3,4-g]chinolin isoliert und

(f) die so erhaltene freie Base gewünschtenfalls in ein Salz überführt und hierdurch ein pharmazeutisch annehmbares Salz dieser Verbindung bildet.

**Patentansprüche für den Vertragsstaat: AT**

1. Verfahren zur Herstellung eines Tautomeren der Formeln Ia oder Ib

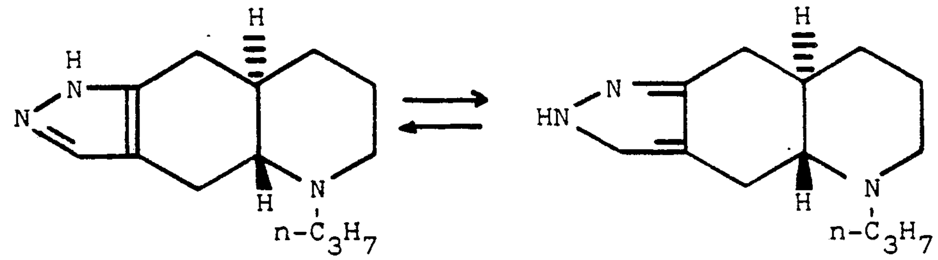

Ia Ib

oder eines pharmazeutisch annehmbaren Salzes hiervon, dadurch gekennzeichnet, daß man

(a) trans-dl-5-n-Propyl-4,4a,5,6,7,8,8a,9-octahydro-1H(und 2H)pyrazol[3,4-g]chinolin mit einem Überschuß einer optisch aktiven Weinsäure der Formel

$$HOOC—(CHOR^2)_2—COOH \qquad (II)$$

worin $R^2$ Wasserstoff oder

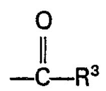 ist,

wobei $R^3$ für $C_1$—$C_3$-Alkyl, Phenyl oder substituiertes Phenyl steht, umsetzt,

(b) das Tartratsalz des so gebildeten trans-Stereoisomeren abtrennt,

(c) das Tartratsalzgemisch durch fraktionierte Umkristallisation in das gewünschte trans-4aR-Tartratsalz überführt,

(d) eine wäßrige Lösug des Tartratsalzes mit einem Überschuß an Base umsetzt, und

(e) die so gebildete freie Base von trans-4aR-5-n-propyl-4,4a,5,6,7,8,8a,9-octahydro-1H(und 2H)pyrazol-[3,4-g]chinolin isoliert und

(f) die so erhaltene freie Base gewünschtenfalls in ein Salz überführt und hierdurch ein pharmazeutisch annehmbares Salz dieser Verbindung bildet.

2. Verfahren nach Anspruch 1, dadurch gekennzeichnet, daß man das Hydrochloridsalz herstellt.

3. Verfahren nach Anspruch 1 oder 2, dadurch gekennzeichnet, daß eine optisch aktive Weinsäure der Formel II verwendet, worin $R^2$ Wasserstoff ist oder $R^3$ für Methyl, Phenyl oder p-Tolyl steht.

4. Verfahren nach Anspruch 3, dadurch gekennzeichnet, daß man als optisch aktive Weinsäure der Formel II D-(−)-S-Weinsäure verwendet.

5. Verfahren nach Anspruch 1, dadurch gekennzeichnet, daß man in Gegenwart eines $C_1$—$C_4$-Alkanols als Lösungsmittel arbeitet.

6. Tautomeres der Formeln Ia oder Ib, dadurch gekennzeichnet, daß es nach dem Verfahren irgendeines der Ansprüche 1 bis 5 hergestellt worden ist.

**Revendications pour les Etats Contractants: BE CH DE FR GB IT LI NL SE**

1. Trans-4aR-5-n-propyl-4,4a,5,6,7,8,8a,9-octahydro-1H(et 2H)pyrazolo[3,4-g]quinoléine de formule Ia ou Ib:

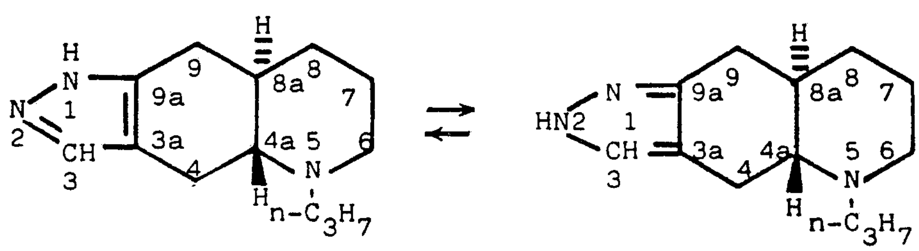

Ia Ib

ou un de ses sels pharmaceutiquement acceptables.

2. Trans-4aR-5-n-propyl-4,4a,5,6,7,8,8a,9-octahydro-1H(et 2H)pyrazolo[3,4-g]quinoléine.

3. Chlorhydrate de trans-4aR-5-n-propyl-4,4a,5,6,7,8,8a,9-octahydro-1H(et 2H)pyrazolo[3,4-g]-quinoléine.

4. Composition pharmaceutique comprenant, comme ingrédient actif, un tautomère de formule Ia ou Ib comme défini dans la revendication 1, ou un de ses sels pharmaceutiquement acceptables, en association avec un support pharmaceutiquement acceptable pour ce tautomère ou ce sel.

5. Tautomère de formule Ia ou Ib ou un de ses sels pharmaceutiquement acceptables selon l'une quelconque des revendications 1 à 3, en vue de l'utiliser comme agent hypotenseur.

6. Procédé de préparation d'un tautomère de formule Ia ou Ib ou d'un de ses sels pharmaceutiquement acceptables selon l'une quelconque des revendications 1 à 3, caractérisé en ce qu'il comprend les étapes qui consistent à:

(a) faire réagir la trans-dl-5-n-propyl-4,4a,5,6,7,8,8a,9-octahydro-1H(et 2H)pyrazolo[3,4-g]quinoléine avec un excès d'un acide tartrique optiquement actif de formule:

$$HOOC—(CHOR^2)_2—COOH \qquad II$$

dans laquelle $R^2$ représente un atome d'hydrogène ou

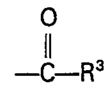

où $R^3$ représente un groupe alkyle en $C_1$—$C_3$, un groupe phényle ou un groupe phényle substitué;

(b) séparer le sel tartrate ainsi formé du trans-stéréoisomère;

(c) soumettre le mélange de sels tartrates à une recristallisation fractionnée pour obtenir le sel tartrate trans-4aR désiré:

(d) faire réagir une solution aqueuse du sel tartrate avec un excès d'une base; puis

(e) isoler la base libre ainsi formée de trans-4aR-5-n-propyl-4,4a,5,6,7,8,8a,9-octahydro-1H(et 2H)-pyrazolo[3,4-g]quinoléine; et

(f) éventuellement salifier la base libre ainsi obtenue pour former un de ses sels pharmaceutiquement acceptables.

**Revendications pour l'Etat contractant: AT**

1. Procédé de préparation d'un tautomère de formule Ia ou Ib:

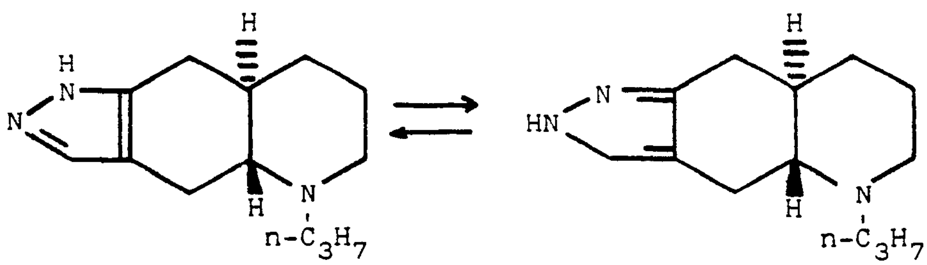

Ia Ib

ou d'un de ses sels pharmaceutiquement acceptables, caractérisé en ce qu'il comprend les étapes qui consistent à:

(a) faire réagir la trans-dl-5-n-propyl-4,4a,5,6,7,8,8a,9-octahydro-1H(et 2H)pyrazolo[3,4-g]quinoléine avec un excès d'un acide tartrique optiquement actif de formule:

$$HOOC—(CHOR^2)_2—COOH \qquad II$$

dans laquelle $R^2$ représente un atome d'hydrogène ou

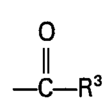

où $R^3$ représente un groupe alkyle en $C_1$—$C_3$, un groupe phényle ou un groupe phényle substitué;

(b) séparer le sel tartrate ainsi formé du trans-stéréoisomère;

(c) soumettre le mélange de sels tartrates à une recristallisation fractionnée pour obtenir le sel tartrate trans-4aR désiré:

(d) faire réagir une solution aqueuse du sel tartrate avec un excès d'une base; puis

(e) isoler la base libre ainsi formée de trans-4aR-5-n-propyl-4,4a,5,6,7,8,8a,9-octahydro-1H(et 2H)-pyrazolo[3,4-g]quinoléine; et

(f) éventuellement salifier la base libre ainsi obtenue pour former un de ses sels pharmaceutiquement acceptables.

2. Procédé selon la revendication 1, dans lequel on forme le sel chlorhydrate.

3. Procédé selon la revendication 1 ou 2, dans lequel l'acide tartrique optiquement actif de formule II est un acide tartrique dans lequel $R^2$ est l'atome d'hydrogène ou $R^3$ est le groupe méthyle, le groupe phényle ou le groupe *p*-tolyle.

4. Procédé selon la revendication 3, dans lequel l'acide tartrique optiquement actif de formule II est l'acide D-(−)-S-tartrique.

5. Procédé selon la revendication 1, dans lequel un alcanol en $C_1$—$C_4$ est présent comme solvant.

6. Tautomère de formule Ia ou Ib préparé par un procédé selon l'une quelconque des revendications 1 à 5.